# EUROPEAN PATENT APPLICATION

(11) **EP 3 696 649 A1**
(43) Date of publication of application: **19.08.2020**
(21) Application number: 18905085.9
(22) Date of filing: 01.06.2018
(51) Int. Cl.: G06F 3/01, G06F 3/0346, A61B 5/024, A61B 5/11

(54) **ELECTRONIC DEVICE AND CONTROL METHOD THEREFOR**

(30) Priority: 08.02.2018 KR 20180015732
(71) Applicant: Samsung Electronics Co., Ltd., Gyeonggi-do 16677 (KR)
(72) Inventor: SLYUSARENKO, Kostyantyn, Kyiv, 01032 (UA); DEGTYARENKO, Illya, Kyiv, 01032 (UA)
(74) Representative: Appleyard Lees IP LLP
(86) International application number: PCT/KR2018/006306
(87) International publication number: WO 2019/156289

(57) **Abstract**

An electronic device is disclosed. The electronic device of the present disclosure comprises: a sensor; a communication unit; and a processor which receives first and second biosignals sensed at a first measurement part from the sensor, receives first and second biosignals sensed at a second measurement part from an external electronic device through the communication unit, synchronizes the second biosignal received from the sensor and the second biosignal received from the external electronic device on the basis of the first biosignal sensed at the first measurement part, and obtains a third biosignal on the basis of the synchronized second biosignals, wherein the rate at which the first biosignal propagates from a predetermined body organ is faster than the rate at which the second biological signal propagates.

## Description

### [Technical Field]

This disclosure relates to an electronic device obtaining a plurality of biosignals and a method for controlling thereof.

### [Background Art]

Development of electronic technology has enabled development and distribution of various types of electronic devices. In particular, a variety of portable electronic devices may sense various biometric information of a user to easily check the health condition.

However, there was a difficulty in obtaining accurate cardiovascular biometric information based on a biosignal sensed at a single measurement point. For example, it was difficult to accurately obtain information such as blood pressure based on the pulse wave travel time sensed at a single measurement point.

Accordingly, there is a need to obtain cardiovascular information based on a biosignal sensed at a plurality of measurement siteparts.

### [Disclosure]

### [Technical Problem]

The disclosure is to address the above-described problems, and an object of the disclosure is to provide biometric information based on a biosignal sensed at a plurality of measurement parts with a specific biosignal as a clock signal.

### [Technical Solution]

According to an embodiment, an electronic device includes a sensor, a communicator, and a processor configured to receive first and second biosignals sensed at a first measurement part from the sensor, receive first and second biosignals sensed at a second measurement part from an external electronic device through the communicator, synchronize the second biosignal received from the sensor and the second biosignal received from the external electronic device based on the first biosignal sensed at the first measurement part, and obtain a third biosignal based on the synchronized second biosignals, and a rate at which the first biosignal propagates from a predetermined body organ may be faster than a rate at which the second biosignal propagates.

A time difference at which a first biosignal in a predetermined first size is measured at different measurement parts may be less than a time difference at which a second biosignal in a predetermined second size is measured at the different measurement parts.

The processor may synchronize a second biosignal sensed at the first measurement part with reference to the first biosignal sensed at the first measurement part, synchronize the second biosignal received from the external electronic device with reference to the first biosignal received from the external electronic device, synchronize the first biosignal received from the external electronic device with reference to the first biosignal sensed at the first measurement part, and the first biosignal may include ballistocardiogram (BCG) and the second biosignal comprises plethysmogram (PPG).

The sensor may include an accelerometer and a PPG sensor, the first biosignal sensed at the first measurement part may be sensed by the accelerometer, and the second biosignal sensed at the first measurement part may be by the PPG sensor.

The processor may obtain the third biosignal by performing a cross-correlation operation for the synchronized second biosignals.

The processor may obtain blood pressure measurement information from the third biosignal using a learning model trained using an artificial intelligence (AI) algorithm.

The third biosignal may include at least one of pulsetransit time (PTT), a respiration rate, a heart rate, or a PPG shape.

The blood pressure measurement information may include at least one of blood pressure, stroke volume, or vascular elasticity.

According to an embodiment, a method for controlling an electronic device includes receiving first and second biosignals sensed at a first measurement part from the sensor and receiving first and second biosignals sensed at a second measurement part from an external electronic device, synchronizing the second biosignal received from the sensor and the second biosignal received from the external electronic device based on the first biosignal sensed at the first measurement part, and obtaining a third biosignal based on the synchronized second biosignals, and a rate at which the first biosignal propagates from a predetermined body organ may be faster than a rate at which the second biosignal propagates.

A time difference at which a first biosignal in a predetermined first size is measured at different measurement parts may be less than a time difference at which a second biosignal in a predetermined second size is measured at the different measurement parts.

The synchronizing may include synchronizing a second biosignal sensed at the first measurement part with reference to the first biosignal sensed at the first measurement part, synchronizing the second biosignal received from the external electronic device with reference to the first biosignal received from the external electronic device, synchronizing the first biosignal received from the external electronic device with reference to the first biosignal sensed at the first measurement part, and the first biosignal may include ballistocardiogram (BCG) and the second biosignal comprises plethysmogram (PPG).

The first biosignal sensed at the first measurement part may be sensed by an accelerometer, and the second biosignal sensed at the first measurement part may be sensed by a PPG sensor.

The obtaining may include obtaining the third biosignal by performing cross-correlation operation for the synchronized second biosignals.

The obtaining may further include obtaining blood pressure measurement information from the third biosignal using a learning model trained using an artificial intelligence (AI) algorithm.

The third biosignal may include at least one of pulsetransit time (PTT), a respiration rate, a heart rate, or a PPG shape.

The blood pressure measurement information may include at least one of blood pressure, stroke volume, or vascular elasticity.

### [Effect of Invention]

According to various embodiments, by synchronizing a plurality of devices using a specific biosignal as a clock signal, accurate biometric information may be obtained.

### [Description of Drawings]

FIG. 1 is a diagram illustrating an electronic system according to an embodiment;
FIG. 2A is a block diagram illustrating a configuration of an electronic device according to an embodiment;
FIG. 2B is a block diagram illustrating a detailed configuration of an electronic device according to an embodiment;
FIG. 3 is a diagram illustrating an operation of receiving a biosignal by an electronic device according to an embodiment;
FIG. 4 is a diagram illustrating a process for performing synchronization of a biosignal according to an embodiment;
FIG. 5 is a diagram illustrating cross-correlation operation according to an embodiment;
FIG. 6 is a diagram illustrating an electronic system according to an embodiment; and
FIG. 7 is a flowchart illustrating a method for controlling an electronic device according to an embodiment.

### [Detailed Description of Embodiments]

After terms used in the present specification are briefly described, the disclosure will be described in detail.

The terms used in the present specification and the claims are general terms identified in consideration of the functions of the various embodiments of the disclosure. However, these terms may vary depending on intention, legal or technical interpretation, emergence of new technologies, and the like of those skilled in the related art. Also, there may be some terms arbitrarily identified by the applicant. Unless there is a specific definition of a term, the term may be construed based on the overall contents and technological common sense of those skilled in the related art.

Since the disclosure may be variously modified and have several embodiments, specific non-limiting example embodiments of the disclosure will be illustrated in the drawings and be described in detail in the detailed description. However, it is to be understood that the disclosure is not limited to specific non-limiting example embodiments, but includes all modifications, equivalents, and substitutions without departing from the scope and spirit of the disclosure. When it is decided that a detailed description for the known art related to the disclosure may obscure the gist of the disclosure, the detailed description will be omitted.

As used herein, the terms "first," "second," or the like may identify corresponding components, regardless of importance of order, and are used to distinguish a component from another without limiting the components.

Singular forms are intended to include plural forms unless the context clearly indicates otherwise. It will be further understood that terms "include" or "formed of' used in the present specification specify the presence of features, numerals, steps, operations, components, parts, or combinations thereof mentioned in the present specification, but do not preclude the presence or addition of one or more other features, numerals, steps, operations, components, parts, or combinations thereof.

A term such as "module," "unit," "part," and so on is used to refer to an element that performs at least one function or operation, and such element may be implemented as hardware or software, or a combination of hardware and software. Further, other than when each of a plurality of "modules," "units," "parts," and the like must be realized in an individual hardware, the components may be integrated in at least one module or chip and be realized in at least one processor (not shown).

Hereinafter, non-limiting example embodiments of the disclosure will be described in detail with reference to the accompanying drawings so that those skilled in the art to which the disclosure pertains may easily practice the disclosure. However, the disclosure may be implemented in various different forms and is not limited to embodiments described herein. In addition, in the drawings, portions unrelated to the description will be omitted, and similar portions will be denoted by similar reference numerals throughout the specification.

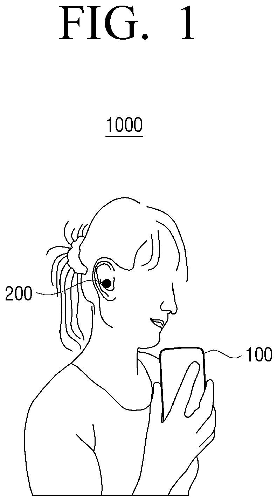

FIG. 1 is a diagram illustrating an electronic system according to an embodiment.

An electronic system 1000 according to an embodiment may include an electronic device 100 and an external electronic device 200.

The electronic device 100 and the external electronic device 200 may be implemented as various portable devices or wearable devices such as a smartphone, a smart watch, an earphone, a smart glass, or the like, and may be implemented as a home appliance, a medical device, a communication device, or the like.

According to an embodiment, the electronic device 100 and the external electronic device 200 may sense a biosignal at different measurement parts. The biosingal sensed at the external device 200 may be transmitted to the electronic device 100, and the electronic device 100 may process the received biosignal and the biosignal sensed by the electronic device 100 together to obtain various biometric information.

For example, as the electronic device 100 and the external electronic device 200 measure the biosignal at different measurement parts, the electronic device 100 may obtain a pulse wave travel time. As the biosignals are measured at a plurality of different measurement parts, a pulse wave travel time passing through different measurement parts spaced apart from each other may be obtained.

However, it is not limited to the pulse wave travel time, and a variety of biosignals may be obtained based on machine learning technologies. As an example, by performing machine learning on the basis of the biosignal sensed by the electronic device 100 and the external electronic device 200 at different measurement parts, various biometric data such as heart rate may be obtained.

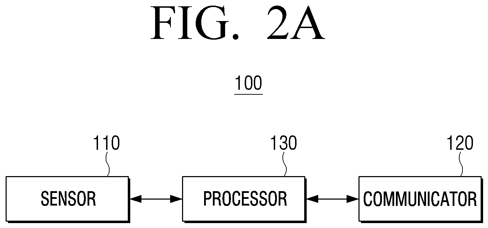

FIG. 2A is a block diagram illustrating a configuration of an electronic device according to an embodiment.

The electronic device 100 according to an embodiment includes a sensor 110, a communicator 120, and a processor 130.

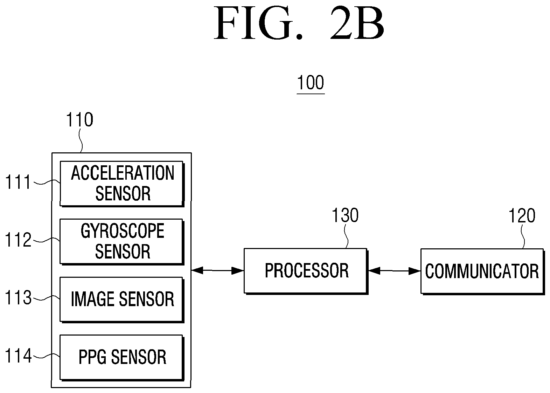

The sensor 110 may sense various biosignals of a user. For example, the sensor 110 may be in contact with a particular portion of the body to sense the biosignal based on a rate of change in accordance with the pulse. The biosignal may include ballistocardiogram (BCG) and plethysmogram (PPG). The sensor 110 may sense the biosignal and transmit the biosignal to the processor 130, and details of the sensor 110 will be described below with reference to FIG. 2B.

The communicator 120 may perform communication with various types of electronic devices. For example, the communicator 120 may communicate through the communication methods such as infrared (IR), wireless fidelity (Wi-Fi), Bluetooth, Zigbee, beacon, near field communication (NFC), wide area network (WAN), Ethernet, IEEE 1394, high definition multimedia interface (HDMI), universal serial bus (USB), mobile high-definition link (MHL), advanced encryption standard (AES) / European broadcasting union (EBU), optical, coaxial, or the like.

For example, the communicator 120 may transmit and receive various biosignals with the external electronic device 200, and may transmit and receive various information necessary for analysis of the biosignal from a server (not shown).

The processor 130 controls overall operations of the electronic device 100.

The processor 130 according to an embodiment may be implemented with a digital signal processor (DSP), a microprocessor, and a time controller (TCON) which process a digital image signal, but embodiments are not limited thereto. The processor 130 may include one or more among a central processing unit (CPU), a micro controller unit (MCU), a micro processing unit (MPU), a controller, an application processor (AP), a communication processor (CP), and an advanced reduced instruction set computing

(RISC) machine (ARM) processor or may be defined as a corresponding term. The processor 130 may be implemented in a system on chip (SoC) type or a large scale integration (LSI) type which a processing algorithm is built therein or in a field programmable gate array (FPGA) type.

The processor 130 according to an embodiment may receive, from the sensor 110, a first biosignal and a second biosignal sensed at a first measurement part, and receive, from the external electronic device 200, the first biosignal and the second biosignal sensed at a second measurement part through the communicator 120.

The processor 130 may receive a first biosignal of which the rate at which the first biosignal propagates from a predetermined body organ is faster than the rate at which the second biosignal propagates. For example, the first biosignal may include BCG, and the second biosignal may include PPG. In this example, the processor 130 may process the second biosignal obtained by the electronic device 100 and the external electronic device 200 based on the first biosignal having a faster propagation rate.

The processor 130 may synchronize the second biosignal received from the sensor 110 and the second biosignal received from the external electronic device 200 based on the first biosignal sensed at the first measurement part.

The processor 130 may perform a first synchronization to synchronize a second biosignal sensed at the first measurement part and a second biosignal received from the external electronic device 200, and perform a second synchronization to synchronize the first biosignal sensed at the first measurement part and the first biosignal received from the external electronic device 200.

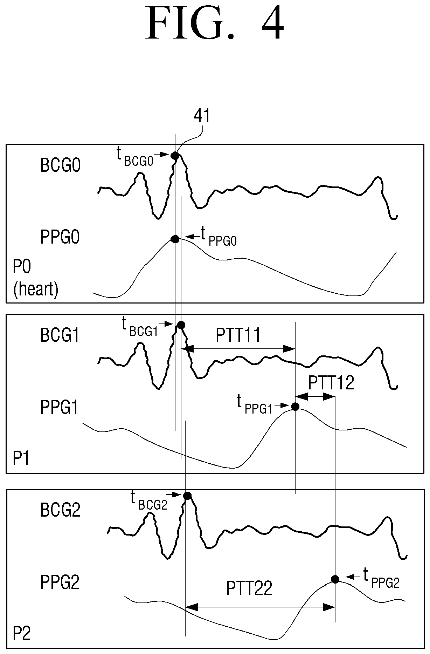

The processor 130 may synchronize the second biosignal and synchronize the first biosignal. This will be further described with reference to FIG. 4.

The processor 130 may obtain a third biosignal based on synchronized second biosignals.

For example, the processor 130 may perform cross-correlation operation for synchronized second biosignals to obtain the third biosignal. By performing the cross-correlation operation, time delay in two waveforms may be identified (or determined).

Accordingly, the processor 130 may determine the time delay of the second biosignals by the cross-correlation operation, and may obtain the third biosignal based on the time delay. For example, the processor 130 may use the BCG and PPG to obtain the third biosignal that includes at least one of a pulsetransit time (PTT), a respiration rate, a heart rate, and a PPG shape.

The processor 130 may obtain cardiovascular information from the third biosignal using a learning model trained using an artificial intelligence (AI) algorithm.

The processor 130 may obtain the cardiovascular information from the third biosignal using shallow learning such as random forest, k-means and a deep learning technology such as a neural networks (NN), recurrent neural networks (RNN), and convolutional neural networks (CNN). In this example, the cardiovascular information may include at least one of blood pressure, stroke volume and vascular elasticity. In one example, the processor 130 may obtain blood pressure information by substituting the PPT to various regression models.

FIG. 2B is a block diagram illustrating a detailed configuration of an electronic device according to an embodiment.

The sensor 110 according to an embodiment may sense various biosignals and may include at least one of an acceleration sensor 111, a gyroscope sensor 112, an image sensor 113, and a PPG sensor 114.

The acceleration sensor 111 and the gyroscope sensor 112 may obtain biosignal based on a quantity of motion of the measurement part and for example, the biosignal may include BCG.

The acceleration sensor 111 and the gyroscope sensor 112 convert the minute vibration of the measurement part according to the heart rate into an electrical signal and transmit the electrical signal to the processor 130, and the processor 130 may identify BCG by analyzing the electrical signal.

The image sensor 113 may obtain at least one of minute vibration and a change of skin color of an imaging area as a sequence of images.

When the image data which captures the minute vibration of the imaging area is transmitted to the processor 130, the image sensor 113 may obtain BCG of the imaging area based on the number of vibrations.

If the image sensor 113 captures the color change of the imaging area as a sequence of images and transmits the captured image data to the processor 130, the processor 130 may obtain the BCG of the imaging area based on the period in which the image data is changed. The image sensor may be implemented with an image sensor such as a charge coupled device (CCD) device, a complementary metal oxide semiconductor (CMOS) device, or the like.

The PPG sensor 114 irradiates the measurement part with a specific light, and if the irradiated light is reflected to the PPG sensor 114, the PPG sensor 114 may transmit information on the reflected light to the processor 130. The processor 130 may analyze the received information to obtain PPG.

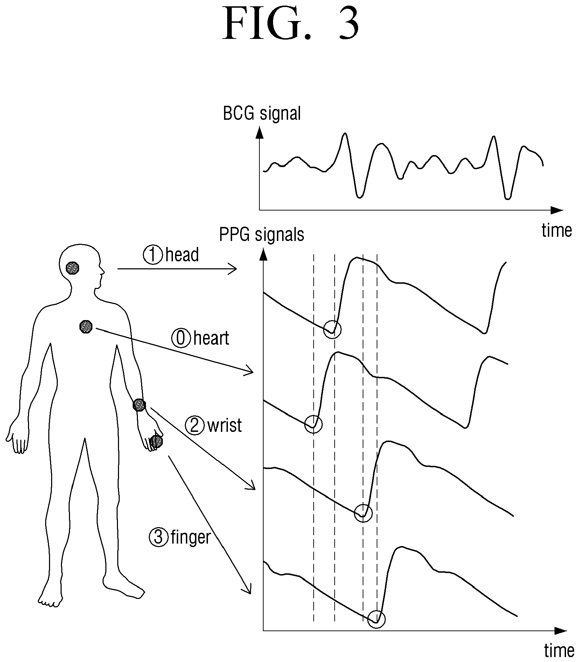

FIG. 3 is a diagram illustrating an operation of receiving a biosignal by an electronic device according to an embodiment.

Referring to FIG. 3, the electronic device 100 and the external electronic device 200 may measure BCG and PPG together at different measurement parts. For example, the measurement part may include, but is not limited to, a chest (heart), a head, a wrist, and a finger.

For example, the electronic device 100 may be implemented as a smart watch, and the external electronic device 200 may be implemented as a smart phone. In this example, the first measurement part may be the wrist, and the second measurement part may be the finger.

The electronic device 100 may receive, from the sensor 110, the BCG and PPG signals at the first measurement part (for example, wrist), and the external electronic device 200 may sense the BCG and PPG signal at the second measurement part (for example, finger) and transmit the same to the electronic device 100.

Referring to FIG. 3, BCG is relatively fast in propagation speed, there is little time shift at each measurement part, whereas PPG is relatively slow in propagation speed and thus, time shift occurs at each measurement part.

Accordingly, a first biosignal including BCG is used as a reference signal, and a time difference of a second biosignal including PPG may be calculated and used as a signal for measuring a third biosignal including the PTT.

FIG. 4 is a diagram illustrating a process for performing synchronization of a biosignal according to an embodiment.

According to an embodiment, the processor 130 may synchronize BCG1, PPG1 sensed at a first measurement part PI, and BCG2, PPG2 sensed at the second measurement part P2. The BCG1 and PPG1 are biosignals sensed by the sensor 110 and received by processor 130, and BCG2 and PPG2 are biosignals sensed by external electronic device 200 and received by the electronic device 100.

The synchronization performed by the processor 130 may include first synchronization and second synchronization.

The processor 130 performs first synchronization by comparing PPG1 and PPG2.

The processor 130 performs the first synchronization using the PPG, since the signal-to-noise ratio (SNR) of the PPG appearing at different measurement parts according to the specific heart rate is greater than the BCG. That is, the processor 130 performs the first synchronization using the PPG first because the distortion generated in the PPG waveform is smaller than the BCG.

The processor 130 may perform the first synchronization having tens of to hundreds of millisecond (msec) by comparing PPG1 and PPG2 having a predetermined number of pulses.

For example, the processor 130 may identify a point at which a value as a result of performing cross-correlation operation of PPG1 and PPG2 reaches a maximum.

The processor 130 may identify a time value of the maximum point as a time delay, and perform synchronization to compensate for the time delay. In this example, the processor 130 performs first synchronization by compensating as much as the time delay for BCG2 and PPG2, respectively.

The processor 130 may remove an error having the size of one second to ten seconds of PPG1 and PPG2 by performing the first synchronization.

However, since the speed of the PPG is slow even though the time delay is compensated by the time delay, it is still possible to have an error of several tens to hundreds of milliseconds.

Accordingly, the processor 130 compares the BCG1 with the BCG2 to perform the second synchronization. That is, the processor 130 may perform raw synchronization (first synchronization) using the PPG1 and the PPG2, and then perform fine synchronization (second synchronization) with less error using the BCG1 and the BCG2, because the propagation speed of the BCG is about 1400 m/s that is greater than about 10 m/s, which is the propagation speed of the PPG.

The processor 130 may lower the error by several tens to hundreds milliseconds and then compare BCG1 and BCG2 to perform the second synchronization.

Since the signal-to-noise ratio of the BCG is lower than the PPG, the processor 130 may perform synchronization using the BCG, because the processor 130 narrows the error to a small range using the first synchronization.

The processor 130 may perform the second synchronization having an error of several tens to 0.5 milliseconds by comparing BCG1 and BCG2 including a predetermined number of pulses.

For example, the processor 130 may identify the point at which the value of the result of performing the cross-correlation operation of the BCG1 and the BCG2 is maximized. The processor 130 may identify the time value of the point at which it is maximized as the time delay, and perform synchronization that compensates for the time delay. In this case, the processor 130 performs synchronization to compensate for the time delay of the BCG2 and the PPG2, respectively.

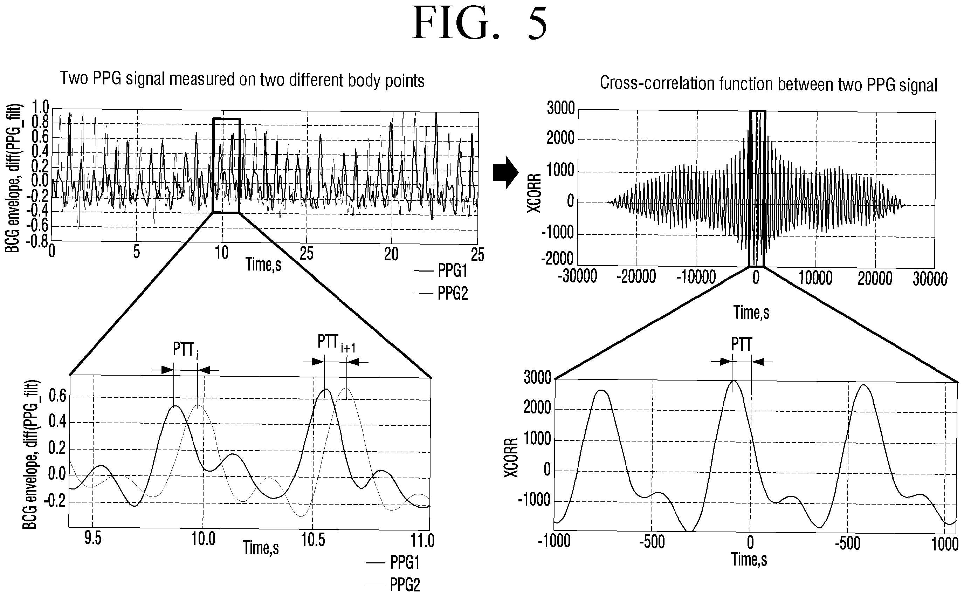

The processor 130 may complete the first synchronization and the second synchronization to eliminate the time delay of BCG1 and BCG2, and use the BCG as a reference signal (clock signal). After performing the first synchronization and the second synchronization, the processor 130 may identify the time delay of the PPG1 and the PTT12 = t_{ppg2} - t_{ppg1} between the first measurement part and the second measurement part. For example, the processor 130 may perform cross-correlation between the PPG1 and the PPG2 to identify a point having a maximum value as the PTT. The process of performing the cross-correlation will be described below with reference to FIG. 5.

The processor 130 may identify PTT between the heart and the first measurement part.

Referring to FIG. 4, the processor 130 may identify a feature point 41 of BCG and PPG. For example, the feature point 41 may include a maximum value.

As illustrated in FIG. 4, the time at which the maximum value of the BCG0 and PPG0 is coincided, as the maximum value 41 of BCG and PPG is formed at a point where the pressure according to cardiac output is maximized.

Accordingly, the processor 130 may identify the time difference at which the feature points of the BCG1 and the PPG1 appear as the PTT11 between the heart and the first measurement part. However, in this example, since the second synchronization of the BCG0 and the BCG1 is not performed, an error within 1 ms may be generated.

As described above, the processor 130 may identify the PTT at the heart and the first measurement part, the heart and the second measurement part, the first measurement part and the second measurement part. The number of measurement parts is not limited thereto, and the greater the number of external electronic devices 200, the greater the various PTT may be identified.

FIG. 5 is a diagram illustrating cross-correlation operation according to an embodiment.

The processor 130 may identify the time delay of both signals by the cross-correlation operation. For example, the processor 130 may identify the time delay of PPG1 and PPG2.

The processor 130 may perform a cross-correlation operation of the PPG1 and the PPG2 to identify the time at which the maximum value appears in the result of performing the operation as a time delay. The cross-correlation operation is an operation method for measuring the similarity of two signals. Since the time at which the maximum value is identified is the time at which the two signals overlap completely, the time at which the maximum value appears may be identified as the delay time.

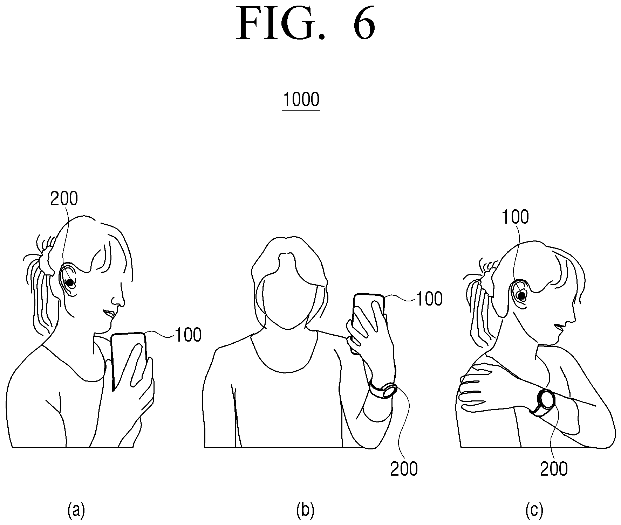

FIG. 6 is a diagram illustrating an electronic system according to an embodiment.

Referring to FIG. 6A, BCG and PPG at each measurement part may be obtained by the combination of a smartphone 100 and an earphone 200.

According to an embodiment, the BCG of the hand portion may be obtained by using the acceleration sensor or the gyroscope sensor of the smartphone 100, and the PPG of the hand portion may be obtained by using the PPG sensor of the smartphone 100. The BCG of the head portion may be obtained by using the acceleration sensor or the gyroscope sensor of the earphone 200, and the PPG of the head portion may be obtained by using the PPG sensor of the earphone 200. The BCG and PPG obtained in the earphone 200 may be transmitted to the smartphone 100 using various communication methods.

According to another embodiment, when the head is photographed using the image sensor of the smartphone 100, the BCG and the PPG of the head portion may be obtained. In this example, the smartphone 100 may obtain the BCG and PPG of the head portion and may simultaneously obtain the BCG and PPG of the hand portion as described above.

Referring to FIG. 6B, BCG and PPG at each measurement part may be obtained by the combination of the smartphone 100 and the smart watch 200.

An acceleration sensor or a gyroscope sensor included in the smart watch 200 may be used to obtain the BCG of the wrist portion, and a PPG of the wrist portion may be obtained using the PPG sensor of the smart watch 200. The smart phone 100 may obtain BCG and PPG of the head portion or hand portion as described above in FIG. 6A.

Referring to FIG. 6C, BCG and PPG at each measurement part may be obtained by the combination of earphone 100 and the smart watch 200. The method of obtaining BCG and PPG by the earphone 100 and the smart watch 200 is the same as the method illustrated in FIGS. 6A and 6B and thus will be omitted.

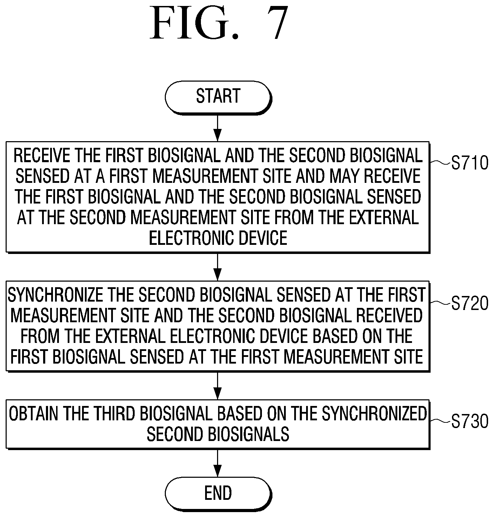

FIG. 7 is a flowchart illustrating a method for controlling an electronic device according to an embodiment.

The electronic device 100 may receive the first biosignal and the second biosignal sensed at a first measurement part and may receive the first biosignal and the second biosignal sensed at the second measurement part from the external electronic device 200 in operation S710.

In this example, the first biosignal may include BCG and the second biosignal may include PPG. However, the embodiment is not limited thereto, and may include various examples in which the propagation speed of the first biosignal is faster than the second biosignal so that the first biosignal may be utilized as a reference signal.

The electronic device 100 may synchronize the second biosignal sensed at the first measurement part and the second biosignal received from the external electronic device based on the first biosignal sensed at the first measurement part in operation S720.

In this example, the electronic device 100 may perform first synchronization and second synchronization. The first synchronization includes synchronization in which the error range is relatively large using the second biosignal with less waveform distortion, and the second synchronization includes synchronization using the first biosignal whose waveform distortion is large but the error range is relatively small. The electronic device 100 may perform pre-processing using the first synchronization, and then perform accurate synchronization within a few milliseconds according to the second synchronization.

The electronic device 100 may obtain the third biosignal based on the synchronized second biosignal in operation S730.

For example, the electronic device 100 may identify a delay time of the second biosignal at a first measurement part and the second biosignal at a second measurement part to obtain the PTT between the first and second measurement parts. However, the embodiment is not limited to the PTT, and various information such as respiration rate, heart rate, the PPG shape, or the like, may be obtained.

The electronic device 100 may obtain biometric information such as blood pressure, cardiac output, and vascular elasticity by using the obtained third biosignal as input data and using shallow learning or deep learning.

At least some configurations of the methods according to the various embodiments as described above may be implemented as an application format installable in an existing electronic device.

At least some configurations among the methods according to the various embodiments as described above may be implemented as software upgrade or hardware upgrade for an existing electronic device.

The various embodiments described above may be performed through an embedded server provided in an electronic device, or an external server of an electronic device.

In addition, one or more embodiments described above may be implemented in a computer readable medium, such as a computer or similar device, using software, hardware, or combination thereof. In some cases, the one or more embodiments described herein may be implemented by the processor itself. According to a software implementation, embodiments such as the procedures and functions described herein may be implemented with separate software modules. Each of the software modules may perform one or more of the functions and operations described herein.

According to the embodiments, computer instructions for performing the processing operations of the apparatus may be stored in a non-transitory computer-readable medium. The computer instructions stored in the non-transitory computer-readable medium may cause a particular apparatus to perform the processing operations on the apparatus according to the one or more embodiments described above when executed by the processor of the particular apparatus.

Non-transitory computer readable medium is a medium that semi-permanently stores data and is readable by the apparatus. Examples of non-transitory computer-readable media may include CD, DVD, hard disk, Blu-ray disk, USB, memory card, ROM, or the like.

While the disclosure has been shown and described with reference to various embodiments, it will be understood by those skilled in the art that various changes in form and details may be made therein without departing from the spirit and scope of the disclosure as defined by the appended claims and their equivalents.

## Claims

1. An electronic device comprising:
a sensor;
a communicator; and
a processor configured to:
receive first and second biosignals sensed at a first measurement part from the sensor,
receive first and second biosignals sensed at a second measurement part from an external electronic device through the communicator,
synchronize the second biosignal received from the sensor and the second biosignal received from the external electronic device based on the first biosignal sensed at the first measurement part, and
obtain a third biosignal based on the synchronized second biosignals,
wherein a rate at which the first biosignal propagates from a predetermined body organ is faster than a rate at which the second biosignal propagates.

2. The electronic device of claim 1, wherein a time difference at which a first biosignal in a predetermined first size is measured at different measurement parts is less than a time difference at which a second biosignal in a predetermined second size is measured at the different measurement parts.

3. The device of claim 1, wherein the processor is further configured to:
synchronize a second biosignal sensed at the first measurement part with reference to the first biosignal sensed at the first measurement part,
synchronize the second biosignal received from the external electronic device with reference to the first biosignal received from the external electronic device,
synchronize the first biosignal received from the external electronic device with reference to the first biosignal sensed at the first measurement part, and
wherein the first biosignal comprises ballistocardiogram (BCG) and the second biosignal comprises plethysmogram (PPG).

4. The electronic device of claim 3, wherein:
the sensor comprises an accelerometer and a PPG sensor,
the first biosignal sensed at the first measurement part is sensed by the accelerometer, and
the second biosignal sensed at the first measurement part is sensed by the PPG sensor.

5. The electronic device of claim 1, wherein the processor is configured to obtain the third biosignal by performing a cross-correlation operation for the synchronized second biosignals.

6. The electronic device of claim 1, wherein the processor is further configured to obtain blood pressure measurement information from the third biosignal using a learning model trained using an artificial intelligence (AI) algorithm.

7. The electronic device of claim 6, wherein the third biosignal comprises at least one of pulsetransit time (PTT), a respiration rate, a heart rate, or a PPG shape.

8. The electronic device of claim 6, wherein the blood pressure measurement information comprises at least one of blood pressure, stroke volume, or vascular elasticity.

9. A method for controlling an electronic device, the method comprising:
receiving first and second biosignals sensed at a first measurement part and receiving first and second biosignals sensed at a second measurement part from an external electronic device;
synchronizing the second biosignal sensed at the first measurement part and the second biosignal received from the external electronic device based on the first biosignal sensed at the first measurement part; and
obtaining a third biosignal based on the synchronized second biosignals,
wherein a rate at which the first biosignal propagates from a predetermined body organ is faster than a rate at which the second biosignal propagates.

10. The method of claim 9, wherein a time difference at which a first biosignal in a predetermined first size is measured at different measurement parts is less than a time difference at which a second biosignal in a predetermined second size is measured at the different measurement parts.

11. The method of claim 9, wherein the synchronizing comprises synchronizing a second biosignal sensed at the first measurement part with reference to the first biosignal sensed at the first measurement part, synchronizing the second biosignal received from the external electronic device with reference to the first biosignal received from the external electronic device, synchronizing the first biosignal received from the external electronic device with reference to the first biosignal sensed at the first measurement part,
wherein the first biosignal comprises ballistocardiogram (BCG) and the second biosignal comprises plethysmogram (PPG).

12. The method of claim 9, wherein the first biosignal sensed at the first measurement part is sensed by an accelerometer, and the second biosignal sensed at the first measurement part is sensed by a PPG sensor.

13. The method of claim 9, wherein the obtaining comprises obtaining the third biosignal by performing cross-correlation operation for the synchronized second biosignals.

14. The method of claim 9, wherein the obtaining further comprises obtaining blood pressure measurement information from the third biosignal using a learning model trained using an artificial intelligence (AI) algorithm.

15. The method of claim 14, wherein the third biosignal comprises at least one of pulsetransit time (PTT), a respiration rate, a heart rate, or a PPG shape.
